Europäisches Patentamt

European Patent Office

Office européen des brevets

(19)

(11) **EP 1 112 752 A2**

(12) **EUROPEAN PATENT APPLICATION**

(43) Date of publication:
**04.07.2001 Bulletin 2001/27**

(51) Int Cl.⁷: **A61L 9/20**

(21) Application number: **00311457.6**

(22) Date of filing: **20.12.2000**

| | |
|---|---|
| (84) Designated Contracting States:<br>**AT BE CH CY DE DK ES FI FR GB GR IE IT LI LU<br>MC NL PT SE TR**<br>Designated Extension States:<br>**AL LT LV MK RO SI**<br><br>(30) Priority: **28.12.1999 JP 37575899**<br><br>(71) Applicant: **TOSHIBA LIGHTING & TECHNOLOGY<br>CORPORATION**<br>**Shinagawa-ku, Tokyo (JP)** | (72) Inventor: **Saitou, Akiko,**<br>**Toshiba Lighting & Technology Corp.**<br>**Tokyo (JP)**<br><br>(74) Representative:<br>**Cheyne, John Robert Alexander Mackenzie**<br>**Haseltine Lake & Co.,**<br>**Imperial House,**<br>**15-19 Kingsway**<br>**London WC2B 6UD (GB)** |

(54) **An odor removing apparatus and a refrigerating apparatus using the same**

(57)    An odor removing apparatus 10.
An ultraviolet ray source 3 radiates ultraviolet rays on a plurality of photocatalytic beads 4. An enclosure 1 allows an airflow and accommodates the light source 3 and the photocatalytic beads 4.

FIG. 1

EP 1 112 752 A2

## Description

The present invention relates to an apparatus for removing or decomposing odor producing ingredients. More particularly, the present invention relates to an apparatus for removing or decomposing odor producing ingredients, which utilizes a photocatalytic substance having a photocatalyst.

An apparatus having a photocatalyst for removing or decomposing odor producing ingredients is known in Japanese Laid Open Patent Application HEI 11-285643 (the '643 application). The odor removing apparatus is provided with an ultraviolet ray lamp, a photocatalytic layer recieving ultraviolet radiation from the lamp, and an enclosure accommodating those elements. The photocatalytic layer comprises titanium oxide and particles of glass beads. The ultraviolet radiation from the lamp is applied to the photocatalytic layer by means of multiple reflecting on the glass beads. Accordingly, photocatalysis effectively occurs, so that the odor ingredients are decomposed and removed.

In this case, since the lamp can not be in the photocatalytic layer, the lamp is inevitably apart from the photocatalytic layer. Accordingly, the energy of the ultraviolet radiation from the lamp reduces slightly, before reaching the photocatalytic layer. Besides, the photocatalytic layer is not able to capture the whole ultraviolet radiation, because the layer does not surround the lamp. As described above, the action of the photocatalysis may be restricted.

Another apparatus having a photocatalyst for removing or decomposing odor ingredients is known in Japanese Laid Open Patent Application HEI 9-129184 (the '184 application). This odor removing apparatus, which has a rare gas discharge lamp, is utilized in a refrigerator. The rare gas discharge lamp can operate in a wide temperature condition. Accordingly, when the rare gas discharge lamp is used in the refrigerator, the lamp efficiency thereof is able to avoid reducing. Since the energy of the ultraviolet radiation from the discharge lamp does not decrease, photocatalysis appropriately occurs. On the other hand, the energy of the lamp is absolutely less than that of a low-pressure mercury vapor discharge lamp.

Furthermore, an odor removing apparatus, which is provided with a Raschig ring and a discharge lamp, is known in Japanese Laid Open Patent Application HEI 9-29065 (the '065 application) . The Raschig ring made of a transmissible material is filled with a photocatalytic substance to be exited by the ultraviolet radiation. In this case, the photocatalytic substance must be selected for improving transmittances of the ultraviolet radiation and airflow. Nevertheless, the shape of the photocatalytic substance is not disclosed in the '065 application for achieving the above.

An object of the invention is to provide an odor removing apparatus, which can improve an ability of removing the odor. Another object of the invention is to provide a refrigerating apparatus using the odor removing apparatus, which can also improve an ability of removing odor at low temperatures.

According to one aspect of the invention, an odor removing apparatus comprises an ultraviolet ray light source, a plurality of photocatalytic beads receiving ultraviolet ray from the light source, and an enclosure, which is able to pass air, accommodating the light source and the photocatalytic beads.

According to another aspect of the invention, an odor removing apparatus comprises an ultraviolet ray discharge lamp provided with an outer bulb filled with gas at less than atmospheric pressure or at atmospheric pressure and a discharge vessel, which contains mercury (Hg) and rare gas, disposed in said outer bulb along the axis thereof and a photocatalytic substance receiving the ultraviolet ray.

According to still another aspect of the invention, a refrigerating apparatus comprises the above-mentioned odor removing apparatus, a photocatalytic substance receiving the ultraviolet ray, an airflow passage holding the odor removing apparatus therein and a refrigerating housing accommodating the odor removing apparatus and the airflow passage.

These and other aspects of the invention will be further described in the following drawings and detailed description of the invention.

In the following, the invention will be described in more detail by way of examples illustrated by drawings in which:

FIGURE 1 is a perspective view of an odor removing apparatus according to an embodiment of the present invention;

FIGURE 2 is a longitudinal cross sectional view of the odor removing apparatus shown in FIG.1;

FIGURE 3 is a graph showing a relationship between quantity of photocatalytic beads and diameter of the photocatalytic beads shown in FIG.1;

FIGURE 4 is a graph showing a relationship between concentration of acetaldehyde gas and operational time of the apparatus;

FIGURE 5 is a cross section of a refrigerating apparatus; and

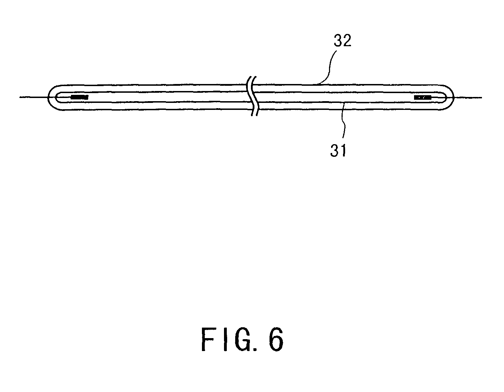

FIGURE 6 is a schematic cross-sectional view of a discharge lamp.

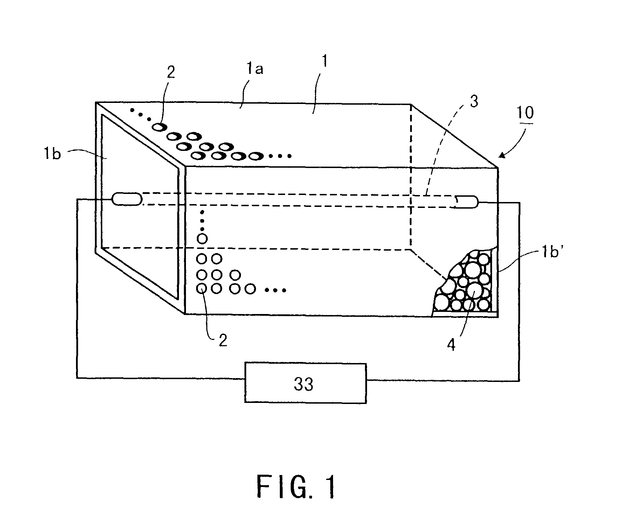

Referring now to FIGS 1 to 5, an embodiment of the invention will be explained.

FIG.1 shows an odor removing apparatus 10 provided with an ultraviolet ray light source 3 to radiate ultraviolet rays, photocatalytic beads 4 to receive the ultraviolet radiation and an enclosure 1 accommodating the light source 3 and photocatalytic beads 4.

The enclosure 1 made of aluminum is shaped in a rectangular body having primary elements defining a plurality of holes and two side plates. The dimensions of the enclosure are respectively about 70 mm in width, 30 mm in height, and 30 mm in depth. The plurality of holes having diameter of about 2 mm are disposed on the primary elements in lines and rows spaced 3 mm from each other. A punching machine preferably forms the holes. Furthermore, since the ultraviolet ray can reflect on an inner surface of the enclosure, the reflected ultraviolet radiation is further applied to the photocatalytic beads 4, so that photocatalysis is effectively activated,

The primary elements may be alternativly made of a mesh having square holes or honeycomb holes. That is, air is able to flow through the primary elements into the photocatalytic beads.

The ultraviolet ray light source 3 may be an ultraviolet ray fluorescent lamp, which is provided with a discharge vessel 31 accommodated in an outer bulb 32 together with rare gas, e.g., xenon, at about 1.3Pa. Moreover, the pressure of the rare gas filling the outer bulb 32 may be atmospheric pressure or less to avoid changing the condition in the discharge vessel. As a result, thermal conductivity between the outer bulb 32 and the discharge vessel 31 lowers, so that the temperature of the discharge vessel is maintained. Accordingly, the lamp can appropriately radiate ultraviolet rays, even if the photocatalytic beads 4 contact the surface of the outer bulb 32.

The fluorescent lamp has preferred dimensions as follows.

| Whole lamp length | 80 mm |
|---|---|
| Outer diameter of the discharge vessel | 1.6 mm |
| Outer diameter of the outer bulb | 2.2 mm |
| The rated lamp power | 0.5 W |

The discharge vessel 31 is filled with a gas, including, for example, mercury (Hg), and rare gas, e. g., argon, krypton or xenon, and a pair of cold cathodes connected to a high frequency inverter circuit 33.

A phosphor layer is coated on the inner surface of the discharge vessel 31. The layer converts ultraviolet radiation of 254 nm generated by the discharge into visible light and ultraviolet radiation within 300 nm to 400 nm. The layer has a luminescent compound, which generates the ultraviolet radiation, is strontium boron oxide activated by europium, e.g., indicated as $SrB_4O_7$: $Eu^{2+}$, which has a peak emission at 368 nm. Furthermore, it may be alkaline earth metal silicate activated by lead, e.g., indicated as follows $(Ba, Sr, Mg)_3 Si_2O_7$: $Pb^{2+}$ or $BaSi_2O_5$: $Pb^{2+}$, alkaline earth metal phosphate activated by europium, e.g., indicated as $(SrMg)_2P_2O_7$: $Eu^{2+}$ or rare earth metal phosphate activated by cerium, e.g., indicated as $YPO_4$: $Ce^{2+}$. Accordingly, the ultraviolet radiation within 300 nm to 400 nm is useful for the photocatalyst activating.

A low-pressure mercury vapor discharge lamp 3 like a fluorescent lamp, a rare gas discharge lamp, or a halogen incandescent lamp may provide the ultraviolet ray light source 3. The fluorescent lamp 3 is located in the center of the enclosure 1, and both sides of the lamp 3 are respectively fixed to the side plates lb, lb' via an insulator, e.g., a rubber or silicone.

About 20g (corresponding to several hundred beads) of the photocatalytic beads 4 fill the enclosure 1. The beads are comprised a spherical body made of silica gel and a photocatalyst layer made of titanium oxide. The photocatalytic beads are preferably manufactured by coating titania sol on the silica gel and drying it. The titanium oxide can be manufactured by other methods such as dipping or spraying titanium oxide on the spherical body, or stacking particles made of titanium oxide on the spherical body. The average diameter of the particles may be more than nano-meter orders, preferably within 50 nm to 3000 nm.

Some of the photocatalytic beads 4 contact the outer bulb 32. The spherical bodies or the photocatalytic beads may not be accurately spherical shape. That is, the bodies or the photocatalytic beads have any shape that does not flaw the outer surface of the fluorescent lamp at a contact surface. The spherical body is preferably made of glass or ceramic, which has higher ultraviolet radiation transmittance.

Since the above mentioned silica gel can also absorb moisture, the silica gel containing moisture effectively promotes the photocatalysis.

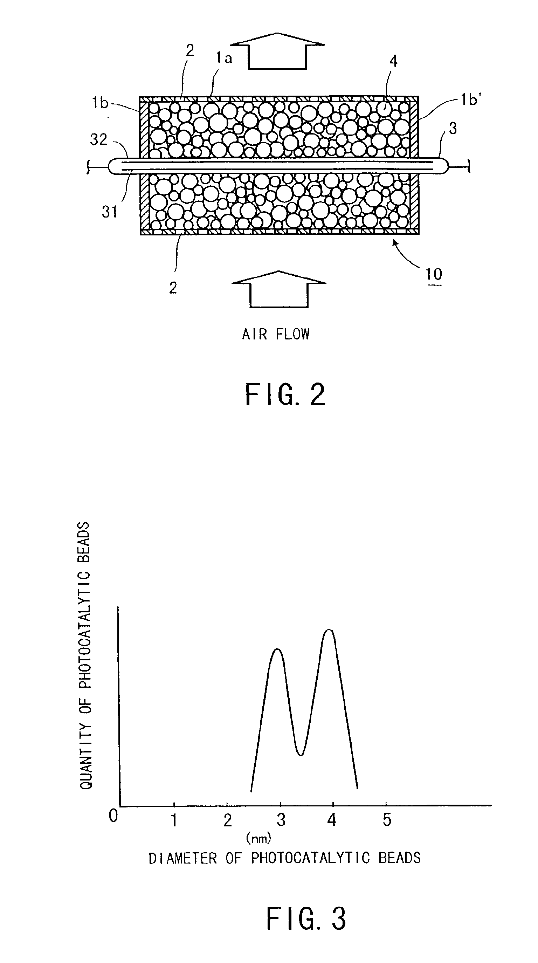

FIG.3 is a graph showing a relationship between a quantity of photocatalytic beads and a diameter of the photocatalytic beads as shown in FIG.1. The graph has two peaks at about 3 mm and 4 mm. In this case, these photocatalytic beads 4 can effectively remove the odor ingredients, because the surface area of the photocatalyst in the enclosure 1 becomes large and also many airflow passages are formed.

In this case, these photocatalytic beads 4 are filled in the enclosure as shown FIG.2, so that many airflow passages are formed through the photocatalytic beads 4 in the enclosure 1. Accordingly, the air can flow from the primary element through the airflow passages to the photocatalytic beads 4.

The above-mentioned photocatalyst is a substance that can convert photo energy into chemical energy. The particles of the metallic oxide constituting the photocatalyst are semiconducting. The semiconductor has a charge band, a conduction band and a band gap therebetween. The band gap has a value of eV inherent to the photocatalyst. For example, when the photocatalyst comprises titanium oxide a sort of semiconductor, its band gap is 3 eV. When light having a wavelength shorter than about 413 nm is applied onto the titanium oxide, electrons in the charge band jump to the conduction band. Thus, the electrons create positive holes $h^+$ where the electrons $e^-$ left. This condition corresponds to an excited condition of the photocatalyst. In the case of titanium oxide, the excited condition is created, when the ultraviolet radiation is applied onto the titanium oxide. In such condition, OH-radical is created around the surface of the photocatalyst, while a hydroxyl group OH reacts on the $h^+$. The $OH^-$ is decomposed from water content $H_2O$ around the photocatalyst. This OH-radical acts to oxidize and decompose the odor ingredients. The substance of photocatalyst may be one or more selected from a group of titanium oxide ($TiO_2$) of anatase structure, zinc oxide (ZnO), tungsten oxide ($WO_3$), iron oxide ($Fe_2O_3$), lanthanum rhodium phosphorus ($LaRhP_3$), iron titanium oxide ($FeTiO_3$), cadmium diiron oxide ($CdFe_2O_4$) and strontium titanium oxide ($SrTiO_3$).

Moreover, titanium oxide or zinc oxide itself may form the photocatalytic beads by means of the known vapor-phase epitaxy method or the cohesive method. In these cases, as the beads comprise the particles of titanium oxide or zinc oxide, ultraviolet radiation can transmit through the particles. Accordingly, photocatalysis occurs even in the particles.

The decomposing operation by means of the odor removing apparatus according to the embodiment of the present invention will be explained hereinafter.

The odor removing apparatus 10 is disposed along airflow passages, which contain odor producing ingredients such as acetaldehyde. The air flows into the photocatalytic beads 4 through one primary element of the enclosure 1, and is exhausted from another primary element. The ultraviolet radiation within 300 nm to 400 nm from the lamp is applied on the photocatalytic beads 4, while ultraviolet radiation passes the airflow passages with reflection or diffusion.

This condition corresponds to an excited condition of the photocatalyst. In the case of titanium oxide, the excited condition is created, when the ultraviolet radiation is applied onto the titanium oxide. An OH-radical is generated around the photocatalyst. This OH-radical acts to oxidize and decompose the above-mentioned odor ingredients, e.g., acetaldehyde ($CH_3CHO$). This oxidation and decomposition occur according to the following chemical formula to eliminate any bad smell.

$$CH_3CHO + OH\text{-radical} \rightarrow CH_3C + H_2O + CO_2$$

Any other odor producing ingredients can also be decomposed oxidation forces of the OH-radical to eliminate the bad smell.

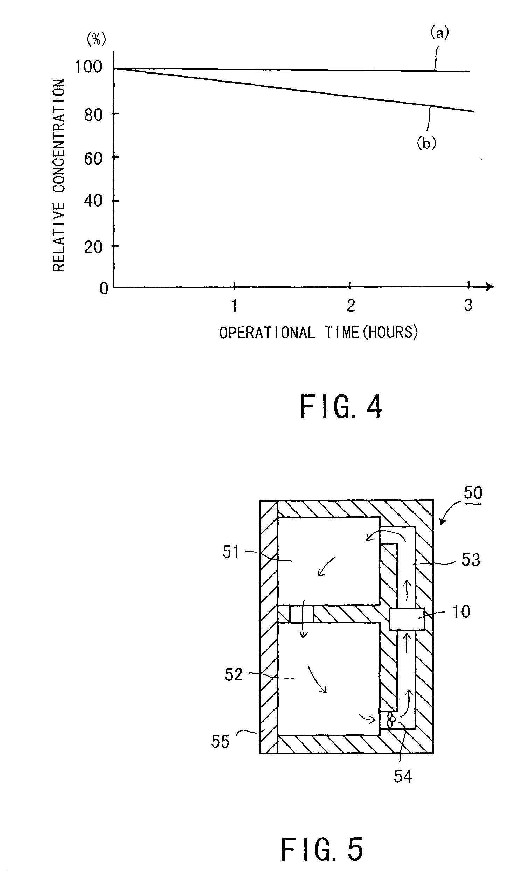

FIG.4 is a graph showing a relationship between concentration of acetaldehyde gas and operational time of the apparatus. The horizontal axis indicates an operational time of the apparatus. The vertical axis indicates a respective concentration of acetaldehyde gas. The Example line (a) designates the relative concentration of acetaldehyde gas referenced to the comparative example line (b). In the comparative example, the apparatus uses a fluorescent lamp without an outer bulb.

The apparatus of the embodiment is disposed in a room having an interior volume of 500 liters. The room is filled with air including acetaldehyde. A blower fan in the room introduces acetaldehyde gas into the apparatus. In the room, the temperature is approximately minus ten degrees centigrade.

In this condition, a gas chromatography is used for operating a quantitative analysis of acetaldehyde gas in every constant time. It is seen that the relative concentration of the example is better than that of the comparative example In the low temperature environment. Because the example can prevent reducing energy of ultraviolet radiation relatively. That is, the outer bulb 32 can maintain a constant mercury vapor pressure of the fluorescent lamp 3, because the outer bulb 32 can prevent dissipation of the heat from the discharge vessel 31.

Now, a refrigerating apparatus including the above-mentioned odor removing apparatus will be explained.

FIG. 5 shows a cross section of the refrigerating apparatus 50. It is provided with a refrigerating room 52, a freezer 51 above the refrigerating room, an airflow passage to communicate them, the odor removing apparatus 10 to be detachably mounted in the airflow passage, a blower fan 54 and an air compressor (not shown). Alternatively, the odor removing apparatus may be disposed in the refrigerating room.

The refrigerating room is separated from the freezer by a partition having another passage allowing airflow. The former passage is disposed at the back of the refrigerating room 52 and the freezer 51, and connects them. Accordingly, the airflow caused by the blower fan 54 circulates in the refrigerating apparatus as shown by arrows in FIG.5.

As the bad smell airflow containing odor producing ingredients circulates, the photocatalytic beads 4 of the odor removing apparatus 10 decompose the odor ingredients.

According to the refrigerating apparatus, since the ultraviolet radiation within 300 nm to 400 nm from fluorescent lamp 3 of the odor removing apparatus 10 is applied on the photocatalytic beads 4, the excited condition of the photocatalyst is created. Accordingly, as mentioned above, OH-radicals act to oxidize and decompose odor ingredients in the airflow.

The fluorescent lamp may light on always, or be controlled by a timer or something to synchronize it. That is, the lamp may be lit, when a door of the refrigerating apparatus opens or shuts. Further, when the compressor or the blow fan operates or stops, the fluorescent lamp may turn on.

As the temperature of the refrigerating room and freezer is less than five degrees centigrade or about five degrees centigrade in the refrigerating apparatus, the airflow and the fluorescent lamp become cold. Thus, generally, the progress of evaporation of mercury (Hg) in a fluorescent lamp is decreased, so that the lamp reduces energy of the ultraviolet radiation. However the fluorescent lamp of this embodiment can prevent lowering the progress of the evaporation. Because the outer bulb 32 of the fluorescent lamp can prevent dissipation of the heat from the discharge vessel 31. Accordingly, the fluorescent lamp can respectively maintain the temperature of the discharge vessel 31 and Hg-vapor pressure thereof. Consequently, even if the odor removing apparatus is used at such a low temperature, the excited condition of the photocatalyst is constantly created. Therefore, the odor removing apparatus can effectively oxidize and decompose the odor ingredients.

Moreover, the oder removing apparatus may be used in an air conditioner or any other apparatus having flowing air.

Other embodiments of the invention will be apparent to those skilled in the art from consideration of the specification and practice of the invention disclosed herein. It is intended that the specification and examples be considered exemplary only, with the true scope and spirit of the invention being indicated by the following claims.

**Claims**

1. An odor removing apparatus comprising:

   an ultraviolet ray light source (3) for radiating ultraviolet rays;
   an enclosure (1) holding said light source (3) and a photocatalytic substance (4), wherein air passes through said enclosure;
   said odor removing apparatus being characterized in that said photocatalytic substance (4) comprises a plurality of photocatalytic beads (4) arranged to receive the ultraviolet rays from said light source (3).

2. An odor removing apparatus according to claim 1, wherein said photocatalytic beads (4) contact said light source (3).

3. An odor removing apparatus according to claim 1, wherein said photocatalytic beads (4) comprise a spherical body made of either glass or silica gel and a photocatalyst comprises titanium oxide, or zinc oxide, coated on the body.

4. An odor removing apparatus according to claim 1, wherein said photocatalytic beads (4) comprise titanium oxide, or zinc oxide.

5. An odor removing apparatus according to claim 1, wherein each photocatalytic bead (4) has a different diameter causing plural peaks of distribution.

6. An odor removing apparatus according to claim 1, wherein said enclosure (1) defines holes (2); and wherein said photocatalytic beads (4) are not able to pass through said holes (2).

7. An odor removing apparatus comprising:

   an ultraviolet ray discharge lamp (3) for radiating ultraviolet rays;
   a photocatalytic substance (4) arranged to receive ultrav iolet rays from said discharge lamp (3);
   an enclosure (1) holding said discharge lamp (3) and said photocatalytic substance (4), wherein air passes through said enclosure (1);
   said odor removing apparatus being characterized in that said ultraviolet ray discharge lamp (3) has an outer bulb (32) filled with gas at pressure less than or equal to atmospheric pressure and a discharge vessel (31) containing mercury (Hg), and a rare gas, said discharge vessel (31) being in said outer bulb (32) along an axis thereof.

8. An odor removing apparatus according to said claim 7, wherein said photocatalytic substance (4) comprises photocatalytic beads; and wherein said photocatalytic beads (4) contact said outer bulb (32) of said ultraviolet ray discharge lamp (3).

9. A refrigerating apparatus, comprising:
   an odor removing apparatus (1), comprising:

   an ultraviolet ray light source (3) for radiating ultraviolet rays,
   a photocatalytic substance (4), and
   an enclosure (1) holding said light source (3) and said photocatalytic substance(4);
   an airflow passage through said odor removing apparatus (10); and
   a refrigerating housing accommodating said odor removing apparatus (10) and said airflow passage;
   said refrigerating apparatus being characterized in that said photocatalytic substance (4) comprises a plurality of photocatalytic beads (4) arranged to receive the ultraviolet rays from said light source (3).

10. A refrigerating apparatus comprising:

    an odor removing apparatus (10) comprising:

    an ultraviolet ray discharge lamp (3) for radiating ultraviolet rays,
    a photocatalytic substance (4) arranged to receive ultraviolet rays from said discharge lamp (3),
    an enclosure (1) holding said discharge lamp (3) and said photocatalytic substance (4), wherein air passes through said enclosure (1);

an airflow passage through said odor removing apparatus (10); and

a refrigerating housing accommodating said odor removing apparatus (10) and said airflow passage;

said refrigerating apparatus being characterized in that said ultraviolet ray discharge lamp (3) has an outer bulb (32) filled with gas at pressure less than or equal to atmospheric pressure and a discharge vessel (31) containing mercury (Hg), and a rare gas, said discharge vessel (31) being in said outer bulb (32) along an axis thereof.

FIG. 1

FIG. 2

AIR FLOW

FIG. 3

QUANTITY OF PHOTOCATALYTIC BEADS

DIAMETER OF PHOTOCATALYTIC BEADS
(nm)

(%)

RELATIVE CONCENTRATION

100 ——————————————————————————— (a)

80 ——————————— (b)

60

40

20

0

         1         2         3

OPERATIONAL TIME (HOURS)

# FIG. 4

# FIG. 5

32

31

FIG. 6